# FASCICULE DE BREVET EUROPEEN

(11) **EP 0 972 512 B1**
(45) Date de publication et mention de la délivrance du brevet: **02.01.2002**
(21) Numéro de dépôt: 99401611.1
(22) Date de dépôt: 28.06.1999
(51) Int. Cl.: A61K 7/48, A61K 7/32, A61K 7/021

(54) **Composition cosmétique comprenant des pigments, un agent antitranspirant et un organopolysiloxane solide élastomérique partiellement réticulé, utilisation d'une telle composition**
Kosmetische Zusammensetzung enthaltend Pigmente, ein Antiperspirant und ein teilvernetztes festes elastomeres Organopolysiloxan, Verwendung einer solchen Zusammensetzung
Cosmetic composition containing pigments, an antiperspirant and a solid elastomeric partially crosslinked organopolysiloxane, use of such a composition

(30) Priorité: 17.07.1998 FR 9809156
(43) Date de publication de la demande: 19.01.2000
(73) Titulaire: L'OREAL, 75008 Paris (FR)
(72) Inventeur: Bara, Isabelle, 75013 Paris (FR)
(74) Mandataire: Brédeville, Odile Marie

(56) Documents cités:
- EP-A- 0 692 238
- EP-A- 0 850 644
- WO-A-98/00104
- WO-A-99/43297
- US-A- 5 654 362

## Description

La présente invention a pour objet une composition cosmétique comprenant au moins un organopolysiloxane solide élastomérique partiellement réticulé et au moins un agent antitranspirant, cette composition pouvant en particulier se présenter sous la forme d'une émulsion, d'un gel, d'une dispersion vésiculaire, et susceptible d'être utilisée pour le maquillage de la peau, des semi-muqueuses, des muqueuses et/ou des phanères.

Les compositions cosmétiques, notamment de maquillage telles que les rouges à lèvres, les anticernes ou les fonds de teint, comprennent généralement des corps gras tels que des huiles et des cires, et une phase particulaire généralement composée de charges et de pigments. Elles peuvent ainsi se présenter, par exemple dans les cas des rouges à lèvres, sous forme de stick ou bâton ou sous forme de pâte souple. Elles sont alors souvent sous la forme d'une composition anhydre. Les compositions de maquillage peuvent également comprendre de l'eau ou une phase hydrophile, et se présenter alors notamment sous forme d'émulsion huile-dans-eau, eau-dans-huile, émulsion multiple, solution ou gel aqueux, notamment lorsqu'il s'agit d'un fond de teint, de crème teintée, de crème de soin ou d'un produit solaire.

Ces compositions, qui sont avant tout des produits de maquillage, ont pour vocation première d'embellir la personne humaine en soulignant par exemple les qualités esthétiques et/ou encore en dissimulant les imperfections du visage.

Selon le maquillage que l'on désire obtenir, léger, discret ou au contraire accentué, on apporte au visage tantôt une nuance proche de la carnation naturelle de la personne, tantôt une couleur plus soutenue qui rehausse le teint naturel : on utilise pour cela les fonds de teint. On peut également accentuer certains reliefs en apportant des tons plus soutenus en utilisant les blushes par exemple. Pour rehausser le regard, on utilise des teintes plus tranchantes couvrant toutes les nuances des couleurs. Enfin, on met les lèvres en valeur en leur apportant des couleurs généralement foncées.

Dans tous les cas, le résultat est obtenu grâce à la présence de pigments et/ou colorants dans ces différentes compositions dont chacune a une fonction bien particulière. Ainsi, la nature des pigments, leur couleur mais surtout leur proportion au sein de chacune de ces compositions sont primordiales pour obtenir la nuance souhaitée et ainsi l'effet désiré.

Il est donc également très important que la répartition de ces pigments et/ou colorants reste constante et homogène au cours du temps, c'est-à-dire même plusieurs heures après l'application. Afin de conserver au visage un maquillage d'ensemble esthétique, il est primordial que les couleurs conférées par chacune des compositions (fond de teint, blush, fard à paupière, rouge à lèvres, etc...) au moment de leur application ne se mélangent pas.

Or, il s'avère bien souvent que l'ensemble du maquillage se détériore peu après l'application des produits : on a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, générées par les mouvements des paupières. On peut également constater l'apparition de parties luisantes ou brillantes ou encore la dilution de la couvrance et de la couleur. Cela peut être dû à de multiples facteurs comme par exemple la chaleur, l'humidité. En particulier, la répartition des couleurs n'est plus aussi homogène. On peut ainsi voir apparaître sur le visage maquillé des parties brillantes et des parties assombries qui vont à l'encontre de l'effet esthétique recherché.

Or les femmes d'aujourd'hui désirent des produits de maquillage qui leur donnent un teint frais et une bonne mine tout au long de la journée tout en étant pratiques d'utilisation. En particulier, elles désirent des produits qui confèrent à leur teint une certaine matité, non seulement à l'application, mais également après plusieurs heures. Ainsi, elles ne désirent pas réappliquer leurs produits plusieurs fois par jour dans le but d'avoir un maquillage net de façon permanente.

Il serait également intéressant de disposer d'un seul produit qui pourrait être utilisé en été, en hiver, dans des pays froids ainsi que dans des pays chauds, en particulier d'un produit qui serait confortable en hiver sans être gras et brillant en été. Un tel produit universel serait très pratique pour les femmes qui voyagent, il permettrait de gagner du temps et serait de moindre coût.

Il subsiste donc le besoin d'une composition cosmétique, notamment de maquillage, qui non seulement confère à la peau une coloration stable et homogène dans le temps tout en ayant de bonnes propriétés cosmétiques mais également qui matifie le teint et ce même plusieurs heures après application sur la peau, en particulier sur le visage.

Or, la Demanderesse a découvert de façon surprenante qu'en introduisant dans des compositions comprenant des pigments un agent antitranspirant en association avec un organopolysiloxane solide élastomérique partiellement réticulé, il était possible de réaliser des compositions de maquillage possédant d'excellentes propriétés cosmétiques, dont la répartition des couleurs est constante au cours du temps et qui matifient le teint même plusieurs heures après l'application sur la peau.

Le document WO99/43297 décrit une composition de maquillage comprenant un organopolysiloxane élastomère, des pigments et un octénylsuccinate d'amidon et d'aluminium.

L'invention a donc pour objet une composition cosmétique conforme à la revendication 1.

Dans l'article "Preventing Makeup Darkening During Usage", Akira Matsueda and Tsuyoshi Ogihara, Cosmetics & Toiletries, Vol. 111, November 1996, il est enseigné que la sécrétion de sébum provoque l'assombrissement des produits de maquillage. Pour remédier à cet inconvénient, il est proposé d'introduire dans les compositions une silice poreuse comprenant un pigment blanc.

Toutefois, il n'a jamais été proposé d'introduire dans un produit de maquillage, en particulier pour le visage, au moins un organopolysiloxane solide élastomérique partiellement réticulé en association avec un agent antitranspirant, notamment dans le but d'améliorer la tenue de la répartition de la couleur après application au cours du temps et de procurer au teint une matité même plusieurs heures après application sur la peau.

Les compositions selon l'invention confèrent au visage une coloration particulièrement stable et dont la répartition reste homogène au cours du temps; elles matifient le teint même plusieurs heures après l'application.

Ces compositions présentent de plus une excellente tenue : elles ne migrent pas dans les plis tels que les paupières ou les rides, on n'observe pas d'accumulation de produit dans certaines parties du visage. Elles transfèrent également très peu sur les supports tels que les tasses ou les vêtements. Elles confèrent à la peau un maquillage homogène, une bonne matité. Elles évitent au visage un aspect poisseux et maintiennent sur ce dernier un état plutôt sec particulièrement recherché dans des climats chauds et humides.

L'invention a encore pour objet l'utilisation, de l'association d'au moins un organopolysiloxane solide élastomérique partiellement réticulé et d'au moins un agent antitranspirant dans une composition cosmétique, en particulier de maquillage, comprenant au moins un pigment afin d'améliorer la tenue de la matité conférée par ladite composition sur la peau.

Un autre objet de l'invention est un procédé de traitement non thérapeutique de la peau et/ou des muqueuses et/ou du cuir chevelu, notamment un procédé de maquillage, consistant à appliquer sur la peau et/ou les muqueuses et/ou sur le cuir chevelu une composition telle que définie ci-dessus.

Les compositions selon l'invention trouvent notamment une application particulièrement intéressante dans le domaine du maquillage de la peau, des muqueuses, des semi-muqueuses, et des phanères. On entend notamment par muqueuse, la partie interne de la paupière inférieure; parmi les semi-muqueuses, on entend plus particulièrement les lèvres du visage; par phanères, on entend les cils, sourcils, cheveux et ongles. Ainsi, l'invention trouve une application toute particulière dans le domaine des produits de maquillage du visage et de la peau, tels que les fonds de teint, les autobronzants ou les produits solaires.

Certes, il est connu d'utiliser des agents antitranspirants dans des compositions telles que les déodorants. Toutefois, personne n'a pensé jusqu'à ce jour à utiliser ces agents en association avec au moins un organopolysiloxane solide élastomérique partiellement réticulé pour des compositions de maquillage sur le visage en raison de problèmes potentiels d'inconfort (irritation, tiraillement, etc...).

Les compositions selon l'invention comprennent au moins un pigment. Par pigments, il faut comprendre des particules blanches ou colorées, minérales ou organiques, insolubles dans le milieu, destinées à colorer et/ou opacifier la composition.

Les pigments peuvent être présents à raison de 0,1-30 % en poids, par rapport au poids total de la composition, et de préférence à raison de 2-15 %. Ils peuvent être blancs ou colorés, minéraux et/ou organiques, de taille usuelle ou nanométrique.

On peut citer, parmi les pigments et les nanopigments minéraux, les dioxydes de titane, de zirconium ou de cérium, ainsi que les oxydes de zinc, de fer ou de chrome, les nanotitanes, le bleu ferrique.

Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.

Ces pigments peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères.

Les compositions selon l'invention comprennent au moins un organopolysiloxane solide élastomérique partiellement réticulé.

Par « élastomérique » on entend un matériau souple, déformable ayant des propriétés viscoélastiques et présentant notamment la consistance d'une éponge ou d'une sphère souple.

Les organopolysiloxanes élastomériques de la composition selon l'invention sont en général partiellement ou totalement réticulés et de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel plus ou moins homogène, en présence de quantités de phase grasse plus élevées. La gélification de la phase grasse par ces élastomères peut être totale ou partielle.

Les élastomères de la composition de l'invention peuvent être véhiculés sous forme de gel constitué d'un organopolysiloxane élastomérique de structure tridimensionnelle, inclus dans au moins une huile hydrocarbonée et/ou une huile siliconée.

Les organopolysiloxanes élastomériques de la composition selon l'invention peuvent être choisis parmi les polymères réticulés décrits dans la demande EP-A-0295886. Selon cette demande, ils sont obtenus par réaction d'addition et de réticulation, en présence d'un catalyseur du type platine, d'au moins :
- (a) un organopolysiloxane ayant au moins deux groupes alcényle en C₁-C₆ par molécule ; et
- (b) un organopolysiloxane ayant au moins deux atomes d'hydrogène liés à un atome de silicium par molécule.

Les organopolysiloxanes élastomériques de la composition selon l'invention peuvent aussi être choisis parmi ceux décrits dans le brevet US 5 266 321. Selon ce brevet, ils sont choisis notamment parmi :
- i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs R₂SiO sur les motifs RSiO_{1,5} allant de 1/1 à 30/1 ;
- ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% en mole lorsque l'organopolysiloxane est non-cyclique et entre 1 et 50% en mole lorsque l'organopolysiloxane est cyclique.

Les organopolysiloxanes objet de l'invention sont par exemple ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels déjà constitués (KSG15, KSG17, KSG16, KSG18 de Shin-Etsu, Gransil SR 5CYC gel, Gransil SR DMF 10 gel, Gransil SR DC 556 gel, SF 1204 et JK 113 de General Electric. On peut aussi utiliser un mélange de ces produits commerciaux.

De façon préférentielle, le ou les organopolysiloxanes sont présents, en matière active, à une concentration allant de 0,1 à 80 % en poids, par rapport au poids total de la composition et de préférence de 0,5 à 20 %, et de préférence encore de 2% à 15%.

De préférence, les compositions selon l'invention ne sont pas transparentes, c'est-à-dire qu'on ne peut pas voir les caractères d'une page de journal à travers la composition. De préférence encore, elles sont colorées et de préférence encore pigmentées.

La composition selon l'invention comprend en outre un agent antitranspirant. Par agent antitranspirant, il faut comprendre un agent limitant la production de la sueur délivré sur la peau. Cet agent antitranspirant n'a pas de fonction colorante. Cet agent peut se présenter sous la forme d'un composé pulvérulent, d'une solution ou d'une dispersion.

Comme agent antitranspirant convenant particulièrement aux compositions selon l'invention, on peut citer :
- les sels d'aluminium tels que l'aluminium chlorohydrate ([Al₂(OH)₅Cl]nH₂O), l'aluminium sesquichlorohydrate ([Al₂(OH)_{4,5}Cl_{1,5}]nH₂O), l'aluminium dichlorohydrate ([Al₂(OH)₄Cl₂]nH₂O, l'aluminium chlorohydrex propylène glycol (PG) ou polyéthylène glycol (PEG) ([Al₂(OH)₅Cl]nH₂O + CH₃CHOHCH₂OH ou H(OCH₂(CH₂)nOH)), l'aluminium sesquichlorohydrex PG ou PEG, l'aluminium PG ou PEG dichlorohydrex, l'aluminium hydroxide (Al(OH)₃nH₂O),
- les chlorhydrates d'aluminium zirconium tels que l'aluminium zirconium trichlorohydrate ([Al_{3,8}Zr(OH)_{12,4}Cl₃]nH₂O), l'aluminium zirconium tétrachlorohydrate ([Al_{3,6}Zr(OH)_{11,6}Cl_{3,2}]nH₂O), l'aluminium zirconium pentachlorohydrate ([Al₈Zr(OH)₂₃Cl₅]nH₂O), l'aluminium zirconium octachlorohydrate ([Al₈Zr(OH)₂₀Cl₈]nH₂O, l'aluminium zirconium trichlorohydrex glycine ([Al_{3,8}Zr(OH)_{12,4}Cl₃]nH₂O + H₂NCH₂COOH), l'aluminium zirconium tétrachlorohydrex glycine, l'aluminium zirconium pentachlorohydrex glycine, l'aluminium zirconium octachlorohydrex glycine,
- le sulfate d'aluminium et de potassium encore appelé alun (KAl(SO₄)₂12H₂O), l'aluminium undecylenoyl collagen aminoacide, le sodium aluminium lactate + sulfate d'aluminium (Al₂(SO₄)₃ + Na₂HAl(OOCCHOHCH₃)₂-(OH)₆), le sodium aluminium chlorhydroxylactate, l'aluminium bromohydrate (Al₂Br(OH)₅nH₂O), le chlorure d'aluminium (AlCl₃6H₂O), les complexes de sel de zinc et de sodium, les complexes de lanthanium et de cérium, le sel d'aluminium de lipoaminoacide (R-CO-NH-CHR'-CO-OAl-(OH)₂ avec R = C₆/C₁₁ et R' = acide aminé).

De préférence, l'agent antitranspirant est un sel d'aluminium et de préférence encore, il est choisi parmi le sulfate d'aluminium et de potassium (alun) et le chlorhydrate d'aluminium.

De préférence, les compositions de l'invention comprennent une quantité efficace d'agent antitranspirant permettant de limiter la migration sur la peau du maquillage après application de ce dernier.

Ainsi, l'agent antitranspirant est généralement présent dans les compositions de l'invention à une teneur allant de 0,1 à 30 %, de préférence de 1 à 10%, en poids, par rapport au poids total de la composition.

Les compositions selon l'invention peuvent comprendre en outre une phase pulvérulente qui peut comprendre, outre les pigments cités plus haut, des nacres et/ou des charges habituellement utilisés dans les compositions cosmétiques. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les nacres peuvent être présentes dans la composition à raison de 0-20 % en poids, de préférence à un taux élevé de l'ordre de 2-15 % en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes dans la composition à raison de 0-20 % en poids, par rapport au poids total de la composition, de préférence 2-10 %, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), les microéponges comme le polytrap (Dow Corning) et les microbilles de résine de silicone (Tospearls de Toshiba, par exemple).

Les compositions de l'invention contiennent en outre un milieu cosmétiquement, hygiéniquement, pharmaceutiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

Les compositions de l'invention peuvent ainsi se présenter sous la forme d'une émulsion huile-dans-eau (H/E), une émulsion eau-dans-huile (E/H), une émulsion multiple ou une solution multiphasée. Elles peuvent également se présenter sous la forme d'un gel anhydre.

Les compositions de l'invention peuvent également se présenter sous la forme d'une dispersion vésiculaire, par exemple sous la forme d'une phase huileuse dispersée dans une phase aqueuse et stabilisée par des liposomes.

Lorsque la composition se présente sous la forme d'une émulsion, elle peut comprendre en outre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

Lorsque la composition se présente sous la forme d'une émulsion H/E, la teneur en agent antitranspirant peut par exemple aller de 1 à 5 % ; lorsque la composition se présente sous la forme d'une émulsion E/H, cette teneur peut aller de 1 à 10 % ; lorsque la composition se présente sous la forme d'un gel aqueux, cette teneur peut aller de 1 à 5 % ; lorsque la composition se présente sous la forme d'un gel anhydre, cette teneur peut aller de 1 à 30 %.

Cette teneur est de préférence choisie par l'homme du métier afin d'obtenir des compositions stables, quelle que soit la forme de ces compositions.

Lorsque la composition selon l'invention comprend une phase aqueuse, cette dernière peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale.

Lorsque la composition selon l'invention comprend une phase grasse, cette dernière peut notamment être constituée de corps gras liquides à 25 °C, tels que des huiles d'origine animale, végétale, minérale ou synthétique.

Lorsque la composition selon l'invention se présente sous la forme d'une émulsion, ladite phase grasse peut comprendre toute huile cosmétiquement acceptable, dans la mesure où ladite huile permet, en mélange avec la phase aqueuse et les éventuels additifs, l'obtention d'une émulsion stable, c'est-à-dire d'une émulsion qui ne casse pas, qui reste sous forme d'une phase unique pendant au moins 24 heures après stockage à 25 °C, sans phénomène de crémage ou de relarguage d'huile.

Les huiles susceptibles d'être employées peuvent éventuellement être volatiles à température ambiante (20-25 °C). On entend par huile volatile, tout composé susceptible de s'évaporer au contact de la peau. De préférence, on utilise des huiles dont le point éclair est suffisamment élevé pour permettre l'utilisation de ces huiles en formulation.

Ces composés volatils peuvent être choisis en particulier parmi les huiles hydrocarbonées et/ou les huiles siliconées, cycliques ou linéaires, seules ou en mélange. On peut ainsi citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (0.01 cm²/s ou 1 cSt). On peut encore citer les alkyltrisiloxanes tels que l'hexylheptaméthyltrisiloxane ou l'octylheptaméthyltrisiloxane.

On peut également citer les huiles volatiles hydrocarbonées, telles que les isoparaffines et notamment l'isododécane.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras et de polyol, en particulier les triglycérides liquides; des alcools; des acétylglycérides; des octanoates, décanoates ou ricinoléates d'alcools ou de polyalcools; des triglycérides d'acides gras; des glycérides, les huiles fluorées et perfluorées;
- leurs mélanges.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.

On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées; leurs mélanges.

Lorsque la composition selon l'invention se présente sous la forme d'une émulsion, elle peut éventuellement comprendre en outre un tensioactif. Comme tensioactif H/E, on peut citer notamment (CTFA) : le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate, le stéarate de triéthanolamine, le stéarate de sodium. Comme tensioactif E/H, on peut citer notamment le mélange polyglycéryl-4 isostéarate/ cétyldiméthicone copolyol/ hexyl laurate, le mélange Mineral oil/petrolatum/ozokérite/glycéryl oléate/alcool de lanoline, les diméthicone copolyols, les cétyl diméthicone copolyols, les diméthicone copolyols substitués en α-ω, c'est-à-dire aux deux extrémités de la chaîne siliconée. De préférence, on utilise les diméthicone copolyols substitués en α-ω.

La composition selon l'invention peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle ou le polyglycéryl 10-décaoléate.

Dans une forme préférée de réalisation, la composition selon l'invention est anhydre et peut se présenter sous la forme d'un gel fluide, d'un stick compact, d'un produit coulé, par exemple dans une coupelle.

En outre, la composition selon l'invention peut comprendre un ou plusieurs agents épaississants.

La composition peut comprendre en outre tout composé complémentaire usuellement utilisé dans le domaine cosmétique, tel que des antioxydants, des parfums, des huiles essentielles, des conservateurs, des actifs cosmétiques ou pharmaceutiques lipophiles ou hydrophiles, des hydratants, des vitamines, des acides gras essentiels, des sphingolipides, des composés auto-bronzants tels que la DHA, des filtres solaires.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Ces composés complémentaires peuvent être présents dans la composition à raison de 0-10 % en poids. Selon leur nature, ils sont présents dans la phase aqueuse ou dans la phase grasse de la composition.

Les compositions selon l'invention peuvent se présenter sous toutes les formes appropriées pour une application topique, notamment sous forme de stick, de lotion, de crème, de lait, de gel anhydre, d'émulsions obtenues par dispersion d'une phase grasse dans une phase aqueuse (H/E) ou inversement (E/H), de consistance liquide ou semi-liquide, voire pâteuse ou solide.

Les compositions selon l'invention peuvent se présenter sous la forme d'un produit cosmétique de maquillage, notamment de la peau et/ou des muqueuses et/ou le cuir chevelu, en particulier pour le corps et/ou le visage, comme par exemple un fond de teint, un fard à joues ou à paupières, un eye-liner, un mascara ou un rouge à lèvres.

Les compositions selon l'invention peuvent constituer tout ou partie d'une composition cosmétique, pharmaceutique ou hygiénique.

Les compositions selon l'invention peuvent être préparées selon les méthodes de préparation classiques des émulsions E/H , H/E, des gels anhydres, ces méthodes étant bien connues de l'homme du métier.

L'invention est illustrée plus en détails dans les exemples suivants. Dans ces exemples, les quantités sont exprimées en pourcentage de poids par rapport au poids total de la composition.

### EXEMPLE 1 :

La Demanderesse a préparé la composition anhydre suivante :

### Fond de Teint :

- mélange de gomme de silicone et de silicone volatile vendu sous la dénomination commerciale "Q2 1401" par Dow Corning 35,46%
- cyclopentasiloxane 14,7%
- organosiloxane réticulé vendu sous la dénomination commerciale "KSG 6" par Shin Etsu 9,8%
- pigments enrobés PDMS 5,63%
- éthanol 3,5%
- chlorhydrate d'aluminium 30% (agent antitranspirant)
- parfum 0,3%

Cette composition a été préparée par mélange des différents composants au sein d'un malaxeur.

Cette composition est stable dans le temps. Après application sur le visage, la composition confère au teint un effet mat, même dans un climat chaud et humide et ce même après plusieurs heures.

La composition est douce à l'application, elle a une bonne tenue et ne génère pas de boulloches sur la peau du visage, en particulier sur le nez, après application.

### EXEMPLE 2 :

La Demanderesse a préparé l'émulsion E/H suivante :
- cyclopentasiloxane 40%
- organosiloxane réticulé vendu sous la dénomination commerciale "KSG 6" par Shin Etsu 20%
- huile de silicone de viscosité 0.05 cm²/s (5 cst) 7,5%
- hydroxyde d'Aluminium (agent antitranspirant) 30% (agent antitranspirant)
- polydiméthylsiloxane vendu sous la dénomination commerciale « Silicone Q₂3225C » par Dow Corning 5 %
- pigment 0,25%
- huile de parléam 0,2%
- acide polyhydoxystéarique/stéarate vendu sous la dénomination commerciale "Solsperse 21000" par ZENECA 0,037%
- MgSO4 0,4%
- propylène glycol 10%
- nacres 1,6%
- conservateur 0,3%
- eau qsp 100%

La composition a été réalisée selon les méthodes classiques de réalisation des émulsions E/H.

La composition est douce à l'application. Elle confère à la peau un teint mat et ce pendant plusieurs heures. Elle ne génère pas de boulloches sur la peau du visage, en particulier sur le nez, après application.

## Revendications

1. Composition cosmétique, notamment de maquillage, comprenant au moins un pigment, **caractérisée en ce qu'**elle comprend en outre au moins un organopolysiloxane solide élastomérique partiellement réticulé et au moins un agent antitranspirant, ledit agent antitranspirant étant choisi parmi:
- l'aluminium chlorohydrate ([Al₂(OH)₅Cl]nH₂O), l'aluminium sesquichlorohydrate ([Al₂(OH)_{4,5}Cl_{1,5}]nH₂O), l'aluminium dichlorohydrate ([Al₂(OH)₄Cl₂]nH₂O, l'aluminium chlorohydrex propylène glycol (PG) ou polyéthylène glycol (PEG) ([Al₂(OH)₅Cl]nH₂O + CH₃CHOHCH₂OH ou H(OCH₂(CH₂)nOH)), l'aluminium sesquichlorohydrex PG ou PEG, l'aluminium PG ou PEG dichlorohydrex, l'aluminium hydroxide (Al(OH)₃nH₂O),
- les chlorhydrates d'aluminium zirconium tels que l'aluminium zirconium trichlorohydrate ([Al_{3,8}Zr(OH)_{12,4}Cl₃]nH₂O), l'aluminium zirconium tétrachlorohydrate ([Al_{3,6}Zr(OH)_{11,6}Cl_{3,2}]nH₂O), l'aluminium zirconium pentachlorohydrate ([Al₈Zr(OH)₂₃Cl₅]nH₂O), l'aluminium zirconium octachlorohydrate ([Al₈Zr(OH)₂₀Cl₈]nH₂O, l'aluminium zirconium trichlorohydrex glycine ([Al_{3,8}Zr(OH)_{12,4}Cl₃]nH₂O + H₂NCH₂COOH), l'aluminium zirconium tétrachlorohydrex glycine, l'aluminium zirconium pentachlorohydrex glycine, l'aluminium zirconium octachlorohydrex glycine,
- le sulfate d'aluminium et de potassium (KAl(SO₄)₂12H₂O), l'aluminium undecylenoyl collagen aminoacide, le sodium aluminium lactate + sulfate d'aluminium (Al₂(SO₄)₃ + Na₂HAl(OOCCHOHCH₃)₂-(OH)₆), le sodium aluminium chlorhydroxylactate, l'aluminium bromohydrate (Al₂Br(OH)₅nH₂O), le chlorure d'aluminium (AlCl₃6H₂O), les complexes de sel de zinc et de sodium, les complexes de lanthanium et de cérium, le sel d'aluminium de lipoaminoacide.

2. Composition selon la revendication 1, **caractérisée en ce que** le pigment est présent à une teneur allant de 0,1 à 30 % en poids, par rapport au poids total de la composition.

3. Composition selon la revendication 2, **caractérisée en ce que** cette teneur va de 2 à 15 %.

4. Composition selon l'une quelconque des revendications 1 à 3, **caractérisée en ce que** les pigments sont choisis parmi les dioxydes de titane, de zirconium ou de cérium, les oxydes de zinc, de fer ou de chrome, les nanotitanes, le bleu ferrique, le noir de carbone, les sels de calcium, de baryum, d'aluminium ou de zirconium, de colorants acides tels que les colorants halogéno-acides, azoïques ou anthraquinoniques.

5. Composition selon l'une quelconque des revendications précédentes, **caractérisée en ce qu'**elle n'est pas transparente.

6. Composition selon l'une quelconque des revendications précédentes, **caractérisée en ce que** l'agent antitranspirant est choisi parmi le sulfate d'aluminium et de potassium et le chlorhydrate d'aluminium.

7. Composition selon l'une quelconque des revendications précédentes, **caractérisée en ce que** l'agent antitranspirant est présent dans la composition à une teneur allant de 0,1 à 30 % en poids, par rapport au poids total de la composition.

8. Composition selon l'une quelconque des revendications précédentes, **caractérisée en ce que** l'organopolysiloxane élastomérique est obtenu par réaction d'addition et de réticulation, en présence d'un catalyseur du type platine, d'au moins :
- (a) un organopolysiloxane ayant au moins deux groupes alcényle en C₁-C₆ par molécule ; et
- (b) un organopolysiloxane ayant au moins deux atomes d'hydrogène liés à un atome de silicium par molécule.

9. Composition selon l'une quelconque des revendications précédentes, **caractérisée par le fait que** l'organopolysiloxane élastomérique est choisi parmi :
- i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs R₂SiO sur les motifs RSiO_{1,5} allant de 1/1 à 30/1 ;
- ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% en mole lorsque l'organopolysiloxane est non-cyclique et entre 1 et 50% en mole lorsque l'organopolysiloxane est cyclique.

10. Composition selon l'une quelconque des revendications précédentes, **caractérisée par le fait que** l'organopolysiloxane élastomérique est présent, en matière active, à une concentration allant de 0,1 à 80 % en poids, par rapport au poids total de la composition et de préférence de 0,5 à 20 %, et de préférence encore de 2% à 15%.

11. Composition selon l'une quelconque des revendications précédentes, **caractérisée par le fait qu'**elle se présente sous la forme d'un produit cosmétique de maquillage du corps et/ou du visage, en particulier d'un fond de teint, d'un fard à joues ou à paupières, d'un eye-liner, d'un mascara ou d'un rouge à lèvres.

12. Utilisation de l'association d'au moins un organopolysiloxane solide élastomérique partiellement réticulé et d'au moins un agent antitranspirant dans une composition cosmétique, en particulier de maquillage, comprenant au moins un pigment afin d'améliorer la tenue de la matité conférée par ladite composition sur la peau

13. Procédé de traitement non thérapeutique de la peau et/ou des muqueuses et/ou du cuir chevelu, notamment un procédé de maquillage, consistant à appliquer sur la peau et/ou les muqueuses et/ou sur le cuir chevelu une composition telle que définie à l'une quelconque des revendications 1 à 11.

## Patentansprüche

1. Kosmetische Zusammensetzung, insbesondere Schminkzusammensetzung, die mindestens ein Pigment enthält, **dadurch gekennzeichnet, daß** sie außerdem mindestens ein teilweise vernetztes, elastomeres, festes Polyorganosiloxan und mindestens ein Antitranspirationsmittel enthält, wobei das Antitranspirationsmittel ausgewählt ist unter
- Aluminiumchlorhydrat ([Al₂(OH)₅Cl]·nH₂O), Aluminiumsesquichlorhydrat ([Al₂(OH)_{4,5}Cl_{1,5}]·nH₂O, Aluminiumdichlorhydrat ([Al₂(OH)₄Cl₂]·nH₂O, Aluminiumchlorhydrex-Propylenglykol (PG) oder -Polyethylenglykol (PEG) ([Al₂(OH)₅Cl]·nH₂O + CH₃CHOHCH₂OH oder H(OCH₂(CH₂)nOH)), Aluminiumsesquichlorhydrex-PG oder -PEG, Aluminium-PG-dichlorhydrex oder Aluminium-PEG-dichlorhydrex, Aluminiumhydroxid (Al(OH)₃·nH₂O),
- den Aluminiumzirconiumchlorhydraten, wie Aluminiumzirconiumtrichlorhydrat (Al_{3,8}Zr(OH)_{12,4}Cl₃]·nH₂O, Aluminiumzirconiumtetrachlorhydrat ([Al_{3,6}Zr(OH)_{11,6}Cl_{3,2}]·nH₂O), Aluminiumzirconiumpentachlorhydrat ([Al₈Zr(OH)₂₃Cl₅]·nH₂O, Aluminiumzirconiumoctachlorhydrat ([Al₈Zr(OH)₂₀Cl₈]·nH₂O, Aluminiumzirconiumtrichlorhydrexglycin ([Al_{3,8}Zr(OH)_{12,4}Cl₃]·nH₂O + H₂NCH₂COOH), Aluminiumzirconiumtetrachlorhydrexglycin, Aluminiumzirconiumpentachlorhydrexglycin, Aluminiumzirconiumoctachlorhydrexglycin,
- Kaliumaluminiumsulft (KAl(SO₄)₂·12H₂O), Aluminiumundecylenoylkollagenaminosäure, Natriumaluminiumlactat + Aluminiumsulfat (Al₂(SO₄)₃ + Na₂HAl(OOCCHOHCH₃)₂-(OH)₆), Natriumaluminiumchlorhydroxylactat, Aluminiumbromhydrat (Al₂Br(OH)₅·nH₂O), Aluminiumchlorid (AlCl₃·6H₂O), die Komplexe eines Zinksalzes und eines Natriumsalzes, die Komplexe von Lanthan und Cer, das Aluminiumsalz von Lipoaminosäuren.

2. Zusammensetzung nach Anspruch 1, **dadurch gekennzeichnet, daß** das Pigment in einem Anteil von 0,1 bis 30 Gew.-%, bezogen auf das Gesamtgewicht der Zusammensetzung, enthalten ist.

3. Zusammensetzung nach Anspruch 2, **dadurch gekennzeichnet, daß** dieser Anteil 2 bis 15 Gew.-% beträgt.

4. Zusammensetzung nach einem der Ansprüche 1 bis 3, **dadurch gekennzeichnet, daß** die Pigmente ausgewählt sind unter Titandioxid, Zirconiumdioxid, Cerdioxid, Zinkoxid, Eisenoxid und Chromoxid, den Nanotitanen, den Eisenblau-Pigmenten, Ruß, den Calciumsalzen, Bariumsalzen, Aluminiumsalzen oder Zirconiumsalzen von sauren Farbstoffen, wie Halogensäure-Farbstoffen, Azofarbstoffen oder Anthrachinonfarbstoffen.

5. Zusammensetzung nach einem der vorhergehenden Ansprüche, **dadurch gekennzeichnet, daß** sie nicht transparent ist.

6. Zusammensetzung nach einem der vorhergehenden Ansprüche, **dadurch gekennzeichnet, daß** das Antitranspirationsmittel unter Kaliumaluminiumsulfat und Alumininiumchlorhydrat ausgewählt ist.

7. Zusammensetzung nach einem der vorhergehenden Ansprüche, **dadurch gekennzeichnet, daß** das Antitranspirationsmittel in der Zusammensetzung in einem Anteil von 0,1 bis 30 Gew.-%, bezogen auf das Gesamgewicht der Zusammensetzung, enthalten ist.

8. Zusammensetzung nach einem der vorhergehenden Ansprüche, **dadurch gekennzeichnet, daß** das elastomere Polyorganosiloxan hergestellt wird durch Additionsreaktion und Vernetzung in Gegenwart eines Katalysators vom Platin-Typ mindestens
(a) eines Polyorganosiloxans, das mindestsens zwei C₁-C₆-Alkenylgruppen pro Molekul aufweist, und
(b) eines Polyorganosiloxans, das mindestens zwei Wasserstoffatome pro Molekül aufweist, die mit einem Siliciumatom verbunden sind.

9. Zusammensetzung nach einem der vorhergehenden Ansprüche, **dadurch gekennzeichnet, daß** das elastomere Polyorganosiloxan ausgewählt ist unter
i) den Polyorganosiloxanen, die Einheiten R₂SiO und RSiO_{1,5} und gegebenenfalls Einheiten R₃SiO_{0,5} und/oder SiO₂ umfassen, worin die Reste R unabhängig Wasserstof, Alkyl, wie Methyl, Ethyl oder Propyl, Aryl, wie Phenyl oder Tolyl, eine ungesättigte aliphatische Gruppe, wie Vinyl, bedeuten, wobei das Gewichtsverhältnis der Einheiten R₂SiO zu den Einheiten RSiO_{1,5} im Bereich von 1/1 bis 30/1 liegt;
ii) den in einem Siliconöl unlöslichen und quellbaren Polyorganosiloxanen, die durch Addition eines Polyorganohydrogensiloxans (1) an ein Polyorganosiloxan (2), das ungesättigte aliphatische Gruppen aufweist, hergestellt werden, wobei die Wasserstoffmenge oder die Menge der ungesättigten aliphatischen Gruppen in (1) bzw. (2) im Bereich von 1 bis 20 Mol-% liegt, wenn das Polyorganosiloxan nicht cyclisch ist, und im Bereich von 1 bis 50 Mol-% liegt, wenn das Polyorganosiloxan cyclisch ist.

10. Zusammensetzung nach einem der vorhergehenden Ansprüche, **dadurch gekennzeichnet, daß** das elastomere Polyorganosiloxan, ausgedrückt als Wirkstoff, in einer Konzentration von 0,1 bis 80 Gew.-%, bezogen auf das Gesamtgewicht der Zusammensetzung, und vorzugsweise in eine Konzentration von 0,5 bis 20 Gew.-% und noch bevorzugter 2 bis 15 Gew.-% enthalten ist.

11. Zusammensetzung nach einem der vorhergehenden Ansprüche, **dadurch gekennzeichnet, daß** sie in Form eines kosmetischen Produkts zum Schminken des Körpers und/oder des Gesichts, insbesondere in Form eines Make-ups, eines Rouge oder Lidschatten, eines Eyeliners, eines Mascaras oder eines Lippenstifts, vorliegt.

12. Verwendung der Kombination aus mindestens einem teilweise vernetzten, elastomeren, festen Polyorganosiloxan und mindestens einem Antitranspirationsmittel in einer kosmetischen Zusammensetzung, insbesondere Schminkzusammensetzung, die mindestens ein Pigment enthält, zur Verbesserung der Beständigkeit der Mattierung, die der Haut durch die Zusammensetzung verliehen wird.

13. Verfahren zur nicht-therapeutischen Behandlung der Haut und/oder der Schleimhäute und/oder der Kopfhaut, insbesondere Schminkverfahren, das darin besteht, auf die Haut und/oder die Schleimhäute und/oder die Kopfhaut eine wie in einem der Ansprüche 1 bis 11 definierte Zusammensetzung aufzutragen.

## Claims

1. Cosmetic composition, in particular a make-up composition, comprising at least one pigment, **characterized in that** it also comprises at least one solid, elastomeric, partially crosslinked organopolysiloxane and at least one antiperspirant, the said antiperspirant being chosen from:
- aluminium chlorohydrate ([Al₂(OH)₅Cl]nH₂O), aluminium sesquichlorohydrate ([Al₂(OH)_{4.5}Cl_{1.5}]nH₂O), aluminium dichlorohydrate ([Al₂(OH)₄Cl₂]nH₂O, aluminium chlorohydrex propylene glycol (PG) or polyethylene glycol (PEG) ([Al₂(OH)₅Cl]nH₂O + CH₃CHOHCH₂OH or H(OCH₂(CH₂)nOH)), aluminium sesquichlorohydrex PG or PEG, aluminium PG or PEG dichlorohydrex and aluminium hydroxide (Al(OH)₃nH₂O),
- aluminium zirconium chlorohydrates, such as aluminium zirconium trichlorohydrate ([Al_{3.8}Zr(OH)_{12.4}Cl₃]nH₂O), aluminium zirconium tetrachlorohydrate ([Al_{3.6}Zr(OH)_{11.6}Cl_{3.2}]nH₂O), aluminium zirconium pentachlorohydrate ([Al₈Zr(OH)₂₃Cl₅]nH₂O), aluminium zirconium octachlorohydrate ([Al₈Zr(OH)₂₀Cl₈]nH₂O, aluminium zirconium trichlorohydrex glycine ([Al_{3.8}Zr(OH)_{12.4}Cl₃]nH₂O + H₂NCH₂COOH), aluminium zirconium tetrachlorohydrex glycine, aluminium zirconium pentachlorohydrex glycine and aluminium zirconium octachlorohydrex glycine,
- potassium aluminium sulphate (KAl(SO₄)₂12H₂O), aluminium undecylenoyl collagen amino acid, sodium aluminium lactate + aluminium sulphate (Al₂(SO₄)₃ + Na₂HAl (OOCCHOHCH₃)₂- (OH)₆), sodium aluminium chlorohydroxylactate, aluminium bromohydrate (Al₂Br(OH)₅nH₂O), aluminium chloride (AlCl₃6H₂O), complexes of zinc salt and of sodium salt, complexes of lanthanum and cerium, and the aluminium salt of lipoamino acids.

2. Composition according to Claim 1, **characterized in that** the pigment is present in a content ranging from 0.1 to 30% by weight relative to the total weight of the composition.

3. Composition according to Claim 2, **characterized in that** this content ranges from 2 to 15%.

4. Composition according to any one of Claims 1 to 3, **characterized in that** the pigments are chosen from titanium dioxide, zirconium dioxide or cerium dioxide, zinc oxide, iron oxide or chromium oxide, nanotitanias, ferric blue, carbon black and the calcium, barium, aluminium or zirconium salts of acidic dyes such as haloacid dyes, azo dyes or anthraquinone dyes.

5. Composition according to any one of the preceding claims, **characterized in that** it is not transparent.

6. Composition according to any one of the preceding claims, **characterized in that** the antiperspirant is chosen from potassium aluminium sulphate and aluminium chlorohydrate.

7. Composition according to any one of the preceding claims, **characterized in that** the antiperspirant is present in the composition in a content ranging from 0.1 to 30% by weight relative to the total weight of the composition.

8. Composition according to any one of the preceding claims, **characterized in that** the elastomeric organopolysiloxane is obtained by addition reaction and crosslinking reaction, in the presence of a platinum-type catalyst, of at least:
- (a) one organopolysiloxane containing at least two C₁-C₆ alkenyl groups per molecule; and
- (b) one organopolysiloxane containing at least two hydrogen atoms linked to one silicon atom per molecule.

9. Composition according to any one of the preceding claims, **characterized in that** the elastomeric organopolysiloxane is chosen from:
- i) organopolysiloxanes comprising units R₂SiO and RSiO_{1.5} and optionally units R₃SiO_{0.5} and/or SiO₂, in which the radicals R, independently of each other, represent a hydrogen, an alkyl such as methyl, ethyl or propyl, an aryl such as phenyl or tolyl, or an unsaturated aliphatic group such as vinyl, the weight ratio of the units R₂SiO to the units RSiO_{1.5} ranging from 1/1 to 30/1;
- ii) organopolysiloxanes which are insoluble and swellable in a silicone oil, obtained by addition of an organohydrogenopolysiloxane (1) and an organopolysiloxane (2) containing unsaturated aliphatic groups, such that the amount of hydrogen or of unsaturated aliphatic groups in (1) and (2), respectively, is between 1 and 20 mol% when the organopolysiloxane is noncyclic and between 1 and 50 mol% when the organopolysiloxane is cyclic.

10. Composition according to any one of the preceding claims, **characterized in that** the elastomeric organopolysiloxane is present, as active material, at a concentration ranging from 0.1 to 80% by weight relative to the total weight of the composition, preferably from 0.5 to 20% and more preferably from 2% to 15%.

11. Composition according to any one of the preceding claims, **characterized in that** it is in the form of a cosmetic make-up product for the body and/or the face, in particular a foundation, a face powder, an eyeshadow, an eyeliner, a mascara or a lipstick.

12. Use of a combination of at least one solid, elastomeric, partially crosslinked organopolysiloxane and of at least one antiperspirant in a cosmetic composition, in particular a make-up composition, comprising at least one pigment, in order to improve the staying power of the matt effect imparted to the skin by the said composition.

13. Nontherapeutic process for treating the skin and/or the mucous membranes and/or the scalp, in particular a make-up process, which consists in applying a composition as defined in any one of Claims 1 to 11 to the skin and/or the mucous membranes and/or the scalp.
